# EUROPEAN PATENT APPLICATION

(11) **EP 4 591 883 A1**
(43) Date of publication of application: **30.07.2025**
(21) Application number: 23868160.5
(22) Date of filing: 19.09.2023
(51) Int. Cl.: A61K 47/04, A61K 9/48, A61K 47/10, A61K 47/14, A61K 47/26, A61K 47/32, A61K 47/36, A61K 47/38, A61K 47/42, A61K 47/44

(54) **LIGHT-SHIELDING HARD CAPSULE CONTAINING MAGNESIUM OXIDE**

(30) Priority: 20.09.2022 WO PCT/JP2022/034937; 21.09.2022 EP 22196956; 28.08.2023 WO PCT/JP2023/030922
(71) Applicant: Capsugel Belgium NV, 2880 Bornem (BE)
(72) Inventor: TAKUBO Takahisa, Sagamihara-shi, Kanagawa 252-0253 (JP); SATO Kaori, Sagamihara-shi, Kanagawa 252-0253 (JP)
(74) Representative: De Clercq & Partners
(86) International application number: PCT/JP2023/033846
(87) International publication number: WO 2024/063039

(57) **Abstract**

PROBLEM TO BE SOLVED: Providing a light-shielding hard capsule shell film and a hard capsule shell used for a pharmaceutical preparation or the like, and a method for producing thereof.

SOLUTION: A hard capsule shell film is produced by a film-forming composition comprising magnesium oxide, which has been safely used as a pharmaceutical product, as a light-shielding agent, instead of titanium oxide, which is considered to have a health problem. The light-shielding hard capsule shell is formed by the film. Since this hard capsule shell can reduce the transmission of light and ultraviolet rays, the stability and quality of any active ingredient filled in the shell can be maintained for a long period of time.

## Description

### [Technical field]

The present invention relates to a light-shielding hard capsule shell film and to a hard capsule shell, wherein the shell is formed from said light-shielding hard capsule shell film, used for pharmaceutical preparations and the like. The present invention also relates to a composition for forming said hard capsule shell comprising a light-shielding agent. The present invention also relates to a method for producing said light-shielding hard capsule shell.

Furthermore, the present invention relates to a light-shielding hard capsule shell film and to a hard capsule shell, wherein the shell is formed from said light-shielding hard capsule shell film, wherein the hard capsule shell film is produced by a film forming composition comprising magnesium oxide as a light-shielding agent, the hard capsule shell film and the hard capsule shell being able to reduce transmission of light and ultraviolet rays and the hard capsule shell thereby being able maintain the stability and quality of any active ingredient filled in said shell for a long period of time.

### [Background technology]

Various colors have been given to various preparations including pharmaceutical products and health food. Some of the purposes of such coloring are: making the preparations highly identifiable; providing the preparations with beautiful appearances; and providing light-shielding property against light in order for quality maintenance. In particular, in a case of a preparation containing an ingredient that is vulnerable to light, a common practice is adding a light-shielding agent of white color into the film forming the shell of a hard capsule shell so that a light-shielding hard capsule shell of white color can be produced. Furthermore, if necessary, various colorants such as various dyes, which can be edible colorants (such as Blue No. 1, Yellow No. 5, Red No. 3, etc.) or various colorants such as pigments (such as iron sesquioxide, aluminum lakes and the like), which can also be edible colorants, are added to give a desired color to the light-shielding film.

Various light-shielding agents that give a light-shielding property to the target material have been known. Among such light-shielding agents, titanium oxide (TiO₂, also referred to as titanium dioxide) is one of the mostfrequently used. Titanium oxide is a white pigment characterized by its excellent whiteness, opacity, and coloring ability as well as its extremely high chemical stability. Hence, titanium oxide has long been used in the field of pharmaceutical products and in the field of cosmetic products.

Some pharmaceutical and cosmetic products have components that are destabilized by titanium oxide or whose decomposition is accelerated by the radicals generated from titanium oxide by UV light. Quality maintenance of such products has always been a problem. In addition, irradiation of light may cause preparations coated with a film containing titanium oxide to undergo peeling of the film and/or the film may become powdery over time, making it difficult to keep the preparations in a coated state. As a result, the external appearance and the handling of such preparations may be impaired. Another matter of concern is that light, oxygen, and/or moisture may cause decomposition of ingredients contained in the preparations.

Then, in producing an opaque or translucent gelatin hard capsule, natural calcium powder (main component is calcium carbonate) with an average particle size of 3 um or less was studied as an opacifying agent that was safer and nutritionally valuable instead of titanium oxide (Patent Document 1).

However, according to the Food Safety Commission of the Cabinet Office, in 2020, the French Food, Environment, Occupational Health and Safety Agency (ANSES) published a written opinion on the identification of nanoparticles, also called nanomaterials, in food and the assessment of consumer health risks. Calcium carbonate is a subject of the evaluation being a food additive containing nanoparticles. Therefore, there are safety issues in the use of calcium carbonate for capsules. (See the website: https://www.fsc.go.jp/fsciis/foodSafetyMaterial/show/syu05400370475)

Regarding carcinogenicity of titanium oxide, International Agency for Research on Cancer (IARC) changed the classification of the carcinogenicity of titanium oxide from Group 3 (not classifiable as to its carcinogenicity to humans) to Group 2B (possibly carcinogenic to humans) in June 2006, when it concludes that the results of animal experiment through inhalation and intratracheal injection of pigment grade and ultra fine TiO₂ gives sufficient evidence to show the carcinogenicity of TiO₂ for animals (Baan R, Staif K, Grosse Y, Secretan B, Ghissassi F, Cogliano V., on behalf of the WHO International Agency for Research on Cancer Monograph Working Group (2006): Carcinogenicity of carbon black, titanium dioxide, and talc; in The Lancet Oncology 2006 Vol. 7, Issue 4, Pages 295-296 https://doi.org/10.1016/S1470-2045(06)70651-9)

Consideration of the above-mentioned problems has led to studies on titanium-oxide free light-shielding film compositions such as the one containing one or more substances selected from water-soluble calcium salts along with a film base agent of a water-soluble cellulose-based polymer (Patent document 2). However, the water-soluble calcium salt described in Patent Document 2 must be used in an amount of 1.0% or less for food according to the usage standard prescribed by "Standards and criteria for food and food additives, etc., 2. Additives". Accordingly, there is a problem that the white coloring density is limited.

On the other hand, as an example of using magnesium oxide as a light-shielding substance, Patent Document 3 discloses a coated solid hypnotic formulation obtained by coating a solid formulation (containing diphenhydramine or an acid addition salt thereof as a medicinal ingredient with sleep and sedative effects) with a film (containing a light shielding material and a water-soluble polymer material). In Patent Document 3, a hard capsule is mentioned as a solid formulation, and magnesium oxide is mentioned as one of the light-shielding materials. However, this formulation does not give light shielding property to the capsule shell itself. Since it is obtained by coating a capsule filled with a drug in the capsule shell with a film comprising a light shielding material and a water-soluble polymer material, there is a drawback that the manufacturing process is complicated.

Patent Document 4 discloses a hard capsule shell having an inner layer of gelatin film and an outer layer comprising 1 to 20% magnesium oxide and a polymer suitable for dipping such as HPMC, gelatin or PVP. However, the layer comprising magnesium oxide is aimed to prevent the gelatin capsule shell from sticking to the oral mucosa and thus it should be on the outmost of the capsule shell.

### [PRIOR ART DOCUMENTS]

### [Patent Document]

[Patent Document 1] JPH07-124462A
[Patent Document 2] WO2004/054619
[Patent Document 3] JP2003-300872A
[Patent Document 4] CN108992417B

### [Summary of the invention]

### [Problems to be solved by the Invention]

While various alternative technologies that may replace titanium oxide as described above have been studied thus far, titanium oxide powder has been widely used as a light-shielding agent in various industrial fields including the food industry and pharmaceutical industry. This is because the very fine particles and the incomparable opacity of titanium oxide provide a sufficient light-shielding ability even with a small amount thereof.

Thus, although it is not desirable for the health food industry to add substances such as titanium oxide that may be harmful to the human body to capsules, it has been difficult to find a suitable alternative to titanium oxide as a light-shielding agent. Therefore, the development of a light-shielding agent to replace titanium oxide has been highly desired.

A further requirement which needs to be met is that any alternative to titanium oxide should provide for hard capsule shell with acceptable mechanical properties. For example, it is desired to have capsules that are sufficiently stable for storage under different relative humidity conditions. Mechanical properties are for example the capsule's fracture and elasticity behavior (e.g. through the tube test), top strength (e.g. through a capsule top compression test), and (pre)lock force strength (e.g. through a (pre)lock force measurement test which is the force required when temporarily connecting the body and cap). They can be important parameters which should be within acceptable ranges. Alternatively for assessing mechanical behavior, several mechanical properties can be measured on films formed by the gelling composition such as: elongation rate (e.g. through a film tensile test), deformation at break (e.g. through a film tensile test), impact resistance (e.g. through an impact energy for fracture test), piercing time by dissolution fluid (e.g. through a piercing time measurement test), or puncture resistance (e.g. through a puncture test). A balance needs to be found between sufficient light shielding characteristics and suitable mechanical properties of the capsules.

For example, when titanium oxide or substitute particles thereof are added to an aqueous film forming polymer solution to produce a capsule shell, the particles make the capsule shell film brittle because the film is only about 100 um thick. There is a risk that the capsule shell will crack in a filling operation. That is, as the particle size of the particles increases or the amount of the particles added increases, the elongation rate of the film decreases remarkably and the capsule shell will easily crack.

In order to avoid the risk of deterioration of the mechanical properties of a hard capsule shell, it is desirable to optimize the particle size and the amount of the light-shielding agent to be added.

### [SOLUTION TO PROBLEMS]

In order to develop a light-shielding agent that can replace titanium oxide, which has been long-awaited in the health food industry, the inventors of the present invention have tackled the above-mentioned technical problems and investigated the possibility of the use of magnesium oxide as a light-shielding agent to be added to a film forming polymer. As a result, it was found that magnesium oxide can realize the beauty of appearance and the light-shielding performance comparable to titanium oxide. It was also found that by combining with other coloring materials, it is possible to perform free coloring at the same time as light-shielding.

By combining various edible tar pigments and natural pigments such as safflower pigments, caramel pigments, and gardenias especially in particular for the health food market, highly safe and beautiful colored light-shielding capsules can be provided.

Moreover, it was found that by using magnesium oxide in form of particles as a light-shielding agent, a hard capsule shell having the above-mentioned essential mechanical property is provided without unduly reducing the elongation rate of the film. Preferably, magnesium oxide with an optimized particle size can be used with optimized amount.

The present invention has been completed based on the above findings and includes the following aspects.
[1] A hard capsule shell wherein the shell is formed by a hard capsule shell film,
   the hard capsule shell film comprising a film forming polymer and magnesium oxide,
   wherein said hard capsule shell film is free from the white-color colorant titanium oxide; and
   wherein the hard capsule shell has only one layer, this one layer is said hard capsule shell film, or
   the hard capsule shell has more than one layer and at least one layer different from the outermost layer is said hard capsule shell film.
[2] The hard capsule shell according to [1], wherein said hard capsule shell film is free from the white-color colorant calcium carbonate.
[3] The hard capsule shell according to [1] or [2], wherein said hard capsule shell film is free from a white-color colorant other than magnesium oxide.
[4] The hard capsule shell according to anyone of [1] to [3], wherein the particle size of magnesium oxide is 50 um or less.
[5] The hard capsule shell according to anyone of [1] to [4], wherein the magnesium oxide has a particle size distribution with a D10 value of from 0.1 to 2.0 um.
[6] The hard capsule shell according to anyone of [1] to [5], wherein the magnesium oxide has a particle size distribution with a D90 value of from 4 to 45 um.
[7] The hard capsule shell according to any one of [1] to [6], wherein the amount of film forming polymer is at least 75 wt. %, with the wt.% being based on the dry weight of the hard capsule shell film.
[8] The hard capsule shell according to any one of [1] to [7], wherein the magnesium oxide is heavy magnesium oxide.
[9] The hard capsule shell according to any one of [1] to [8], wherein the amount of magnesium oxide is from 0.25 to 20 wt. %, with the wt.% being based on the dry weight of the film forming polymer.
[10] The hard capsule shell according to any one of [1] to [9], wherein the amount of magnesium oxide is from 2 to 10 wt. %, with the wt.% being based on the dry weight of the film forming polymer.
[11] The hard capsule shell according to any one of [1] to [10], wherein the amount of magnesium oxide is from 5 to 8 wt. %, with the wt.% being based on the dry weight of the film forming polymer.
[12] The hard capsule shell according to any one of [1] to [11], wherein the film forming polymer is selected from the group consisting of gelatin, polyglutamic acid, cellulose derivative, pullulan, starch, dextrin, agar, chitosan, acid-treated crushed yeast, polyvinyl alcohol (PVA), PVA-methylmethacrylate copolymer, polyvinylpyrrolidone (PVP).
[13] The hard capsule shell according to any one of [1] to [12], further comprising a plasticizer.
[14] The hard capsule shell according to [13], wherein the plasticizer is selected from the group consisting of xylitol, fructose, sorbitan fatty acid ester, triethyl citrate, glycerin, sorbitol, mannitol, trehalose, vegetable oil, medium chain fatty acid triglyceride, triacetin, phthalates, phytosterol, propylene glycol, polysorbate and polyethylene glycol.
[15] The hard capsule shell according to [13] or [14], wherein the amount of the plasticizer is from 0.01 to 10 wt. %, with the wt. % being based on the dry weight of the film forming polymer.
[16] The hard capsule shell according to any one of [14], wherein the plasticizer is sorbitan fatty acid ester.
[17] The hard capsule shell according to [16], wherein the plasticizer is sorbitan monolaurate.
[18] The hard capsule shell according to any one of [1] to [17], which is colored by adding a colorant wherein the colorant have a color other than white.
[19] A method for preparing a hard capsule shell according to claim 1, wherein said hard capsule shell film comprising a film forming polymer and magnesium oxide is prepared by dip molding.
The unit «um» means the same as the unit «µm», that is «micrometer», if not otherwise stated.

The hard capsule shell of the present invention is intended to be used in the fields of pharmaceuticals and food. Therefore, the materials used for producing the hard capsule shell are limited to those that can be safely used in the fields of pharmaceuticals and food.

Magnesium oxide occurs naturally as the colorless, crystalline mineral periclase. It is produced either as a bulky white powder (light) or a relatively dense white powder (heavy) by heating magnesium hydroxide or carbonate. Heating these magnesium salts under moderate conditions (400° to 900 °C for a few hours) produces the so-called light magnesium oxide. Heating the salts under more rigorous conditions (such as 1200 °C for 12 hours) produces the so-called heavy magnesium oxide. Light magnesium oxide is converted to heavy magnesium oxide by sustained heating at high temperatures.

Magnesium oxide used in the present invention is preferably produced at a calcining temperature of at least 700° C, preferably at least 800° C, more preferably at least 900° C, even more preferably at least 925°C. Particular embodiments of calcining temperature are 950°C and 1'300°C.

Magnesium oxide used in the present invention is widely used as an antacid and laxative in the field of medicine. It is also used as magnesium fortification, supplements, dietary supplements and the like in the food field. However, until now, it has never been used as a light-shielding agent for a hard capsule shell, that is, as a light-shielding agent for the hard capsule shell film which forms the shell of the hard capsule shell.

Said magnesium oxide in said hard capsule shell film, which comprises said film forming polymer and said magnesium oxide, is used as light-shielding agent and/or as whitening agent,

The magnesium oxide used as a light-shielding agent for the hard capsule shell film preferably has a particle size of 50 um or less, or 45 um or less, or 40 um or less, or 35 um or less, or 30 um or less, or 25 um or less, or 20 um or less, or 15 um or less. If it exceeds 50 um, the film strength is reduced.

The particle size distribution may also be characterized by a D10, and/or a D90 value; the D10 / D90 value is the particle size where 10% / 90% of the particles have a particle size smaller than the D10 / D90 value. In this case, % means cumulative volume. The values D50 and D100 are defined respectively: the D50 or D100 value is the particle size where 50% or 100% respectively of the particles have a particle size smaller than the D50 or D100 value.

In one embodiment, the magnesium oxide has a particle size distribution with a D10 value of 2.0 um or less, preferably of 1.5 um or less, more preferably of 1.0 um or less. In one embodiment, the magnesium oxide has a particle size distribution with a D10 value of 0.1 um or more. In one embodiment, the magnesium oxide has a particle size distribution with a D10 value of from 0.1 to 2.0 um, preferably of from 0.1 to 1.5 um, more preferably of from 0.1 to 1 um.

Preferably, the magnesium oxide has a particle size distribution with a D90 value of 45 um or less, preferably of 40 um or less, more preferably of 35 um or less, even more preferably of 30 um or less, especially of 25 um or less, more especially of 20 um or less, even more especially of 15 um or less, in particular of 10 um or less, more in particular of 9 um or less.
In another embodiment, the magnesium oxide has a particle size distribution with a D90 value of 20 µm or less, preferably 15 µm or less, more preferably 10 µm or less, even more preferably 7.5 µm or less, especially 5 µm or less, more especially 2 µm or less, even more especially 1.5 µm or less, in particular 1.25 µm or less.
Preferably, the magnesium oxide has a particle size distribution with a D90 value of 4 um or more.
In another preferred embodiment, the magnesium oxide has a particle size distribution with a D90 value of 0.3 um or more.

Preferably, the magnesium oxide has a particle size distribution with a with a D90 value of from 4 to 45 um, preferably of from 4 to 40 um, more preferably of from 4 to 35 um, even more preferably of from 4 to 30 um, especially from of from 4 to 25 um, more especially of from 4 to 20 um, even more especially of from 4 to 15 um, in particular of from 4 to 10 um, more in particular of from 4 to 9 um.
In another preferred embodiment, the magnesium oxide has a particle size distribution with a D90 value of from 0.3 to 20 µm, preferably from 0.3 to 15 µm, more preferably from 0.3 to 10 um, even more preferably from 0.3 to 7.5 um, especially from 0.3 to 5 um, more especially from 0.3 to 2 um, even more especially from 0.3 to 1.5 um, in particular from 0.3 to 1.25 um.

Any of the given ranges for the D10 values may be combined with any of the given ranges for the D90 values.

In one embodiment, the magnesium oxide has a particle size distribution with a D10 value of from 0.1 to 2.0 um; and with a D90 value of from 4 to 45 um.

In another embodiment, the magnesium oxide has a particle size distribution with a D10 value of from 0.1 to 2.0 um and with a D90 value of from 0.3 to 20 um.

Preferably, the magnesium oxide has a particle size distribution with a D10 value of from 0.1 to 1.5 um and with a D90 value of from 5.0 to 8.0 um.

In another preferred embodiment, the magnesium oxide has a particle size distribution with a D10 value of from 0.1 to 1.0 um and with a D90 value of from 0.3 to 7.5 um.

The particle size distribution is measured as follows:
A dispersion comprising 40 wt.% of magnesium oxide in water is prepared with an Ultra Turrax high-speed stirrer at 6'600 rpm for 15 minutes. Water is added to dilute it to about 1 x 10⁻⁴ g MgO / L. The particle size distribution is measured by Shimadzu particle size distribution analyzer SALD-2000J at a wave length of 680 nm. Both light magnesium oxide and heavy magnesium oxide can be used as a light-shielding agent as well, with heavy magnesium oxide being preferred.
Exemplary grades of magnesium oxide are given in Table 1.

**[Table 1]**

| MgO Product | MS-0 "MgO, Food grade" (TOMITA Pharmaceutical CO.,Ltd, Japan) | MS-2 |
|---|---|---|
| Calcining Temp. | 600 °C | 1300 °C |
| Particle size distribution [um] | | |
| D10 | 0.801 | 0.8 |
| D50(*) | 2.577 | 1.5 |
| D90 | 5.739 | 4.1 |
| D100(*) | 11.1 | 25 |

When preparing film-forming polymer solutions comprising magnesium oxide, it is desirable to disperse the magnesium oxide in water as concentrated as possible in order to have maximum flexibility. Therefore, 20% and 40% dispersions of MS-0 and MS-2 were prepared, and changes in viscosity of them were measured for 8 days.

The dispersion was prepared as follows:
A mixture of the magnesium oxide product and water was dispersed using a homogenizer at 10'000 rpm for 10 minutes, and the mixture was stirred with a spatula to prepare a dispersion. The viscosity of the dispersion was measured at the time of preparation, after 1 day, and after 8 days with a Brookfield digital viscometer (BROOKFIELD DV2T). Table 2 shows the results.

**[Table 2]**

| | Viscosity [mPas] | | | |
|---|---|---|---|---|
| | MS-0 | | MS-2 | |
| | 20% | 40% | 20% | 40% |
| T=0 day | 28 | 1263 | 431 | 979 |
| T=1 day | 67 | 19650 | 296 | 1161 |
| T=8 days | 1285 | (*) | 13 | (**) |

| | | | | |
|---|---|---|---|---|
| (*) hardened like gypsum (**) still can be stirred | | | | |

As soon as MS-0 is made into a 40% dispersion, the viscosity of the dispersion rises and it hardens like gypsum. Therefore, it is necessary to add the 40% MS-0 dispersion immediately after preparation to the film-forming polymer solution to reduce the MgO concentration and avoid solidification. Since MS-2 does not have such a phenomenon of solidification, it is possible to store a high-concentration dispersion like 40% and use it for preparation of the film-forming polymer solution when necessary; MS-2 therefore gives greater flexibility.

Film forming polymers used in the present invention include those that can be used in pharmaceuticals and food. For example, polyvinyl alcohol (PVA), PVA-methyl, methacrylic acid copolymer, polyvinylpyrrolidone (PVP), proteins such as gelatin and polyglutamic acid, which are the usual raw materials for capsule films, polysaccharides such as cellulose derivatives, pullulan, starch, dextrin, agar, chitosan, acid-treated crushed yeast, and the like can be used. The film forming polymer is not limited to the above, as long as it is a film forming polymer.
Preferred film forming polymers are gelatin, cellulose derivatives or pullulan.

Examples of the cellulose derivatives include non-enteric cellulose derivatives and enteric cellulose derivatives. Non-enteric cellulose derivatives include water-soluble cellulose ether, a water-soluble cellulose ether is preferably a water soluble cellulose ether in which a part of the hydrogen atom of the hydroxyl group of cellulose is replaced with an alkyl group, such as C₁₋₄ alkyl group, with a hydroxyalkyl group, such as C₁₋₄ hydroxyalkyl group, or with a combination thereof; examples include hydroxy lower alkyl celluloses such as methyl cellulose, hydroxypropylcellulose (HPC) and hydroxyl-lower alkyl alkyl celluloses such as hydroxyethylmethylcellulose, hydroxyethylethylcellulose, and hydroxypropylmethylcellulose (HPMC); among them, hydroxypropyl methylcellulose (hypromellose, HPMC) is preferred. Examples of HPMC, which can be suitably used, are:
HPMC 2910 containing 7.0 to 12.0% hydroxypropoxy group and 28.0 to 30.0% methoxy group;
HPMC 2906 containing 4.0 to 7.5% hydroxypropoxy group and 27.0 to 30.0% methoxy group;
HPMC 2208 containing 4.0 to 12.0% of hydroxypropoxy group and 19.0 to 24.0% of methoxy group;
HPMC 1828 containing about 23.0 to 32.0% hydroxypropoxy group and about 16.5 to 20.0% methoxy group, and mixtures thereof.

In the instant invention, the HPMC methoxy and hydroxypropoxy contents are expressed according to US Pharmacopeia.

HPMC 2910, HPMC 2906 and HPMC 2208, for examples, are listed in USP43-NF38. Various HPMC grades may be used alone or in combination. Preferred HPMC is HPMC 2906, HPMC 2910 and mixtures thereof.

Enteric cellulose derivatives include enteric polymers such as hydroxypropyl methylcellulose acetate succinate (HPMCAS), polyacrylic acid copolymers, carboxymethyl ethyl cellulose, hypromellose phthalate ester (HPMCP) and cellulose acetate phthalate (CAP).

In a preferred embodiment, cellulose derivatives are selected from the group consisting of hypromellose, hydroxypropyl methylcellulose acetate succinate (HPMCAS), carboxymethyl ethyl cellulose, hypromellose phthalate ester (HPMCP) and cellulose acetate phthalate (CAP), with hypromellose being more preferred, also with its embodiments described herein.

Gelatin may include alkaline treated gelatin extracted from bovine or porcine bone or acid treated gelatin extracted from bovine skin, porcine skin, or fish scales. A mixture of the above alkali-treated gelatin and acid-treated gelatin can be used, too.

The amount of the film forming polymer in said film is preferably at least 75 wt. %, or at least 77.5 wt.%, or at least 80 wt.%, or at least 82.5 wt.%, or at least 85 wt.%, or at least 87.5 wt.% or at least 90 wt.%, or at least 92.5 wt.%, or at least 95 wt.%, or at least 97.5 wt.%, with the wt.% being based on the dry weight of the film.

The amount of the film forming polymer is preferably from 83.3 to 99.75 wt. %, preferably from 90.9 to 98.0 wt. %, more preferably from 92.6 to 95.2 wt. %, with the wt. % being based on the dry weight of the film.

Magnesium oxide can be added in an amount of from 0.25 to 20 wt. %, preferably from 2 to 10 wt. %, more preferably from 5 to 8 wt. %, based on the weight (dry weight) of the film forming polymer. If it is more than 20 wt. %, the film becomes brittle and easily cracked, and if it is less than 0.25 wt. %, whitening does not occur, or the color is too light and light shielding cannot be expected.

In an embodiment of the invention, said hard capsule shell film and thereby said hard capsule shell consist of the film forming polymer and magnesium oxide only, except for water in form of residual moisture.

In case that said hard capsule shell film and thereby said hard capsule shell consist of the film forming polymer and magnesium oxide only, except for water in form of residual moisture, possible ranges for film forming polymer and magnesium oxide based on weight of dry film or dry capsule shell respectively are:
from 83.33 to 99.75 wt.% of film forming polymer and
from 0.25 to 16.67 wt.% of magnesium oxide, preferably
from 90.91 to 98.04 wt.% of film forming polymer and
from 1.96 to 9.09 wt.% of magnesium oxide, more preferably
from 92.59 to 95.24 wt.% of film forming polymer and
from 4.76 to 7.41 wt.% of magnesium oxide.

The addition of magnesium oxide tends to reduce the elongation rate of the film, making it more susceptible to impact. The addition of a plasticizer can improve the elongation rate of the film.

Some examples of the above plasticizers include xylitol, fructose, sorbitan fatty acid ester, triethyl citrate, glycerin, sorbitol (sorbitol / sorbitan solution), mannitol, trehalose, vegetable oil (sesame oil, castor oil), medium chain fatty acid triglyceride, triacetin, and phthalic acid esters (phthalic acid dioctyl), phytosterol, propylene glycol, polysorbate, polyethylene glycol (macrogol) and the like. A preferable plasticizer is sorbitan fatty acid ester, more preferably sorbitan monolauric acid ester (CAS 1338-39-2, sorbitan monolaurate).

The amount of the plasticizer is from 0.01 to 10 wt. %, preferably from 0.05 to 5 wt. %, more preferably from 0.05 to 2.5 wt. %, even more preferably from 0.3 to 1.5 wt. %, based on the weight (dry weight) of the film forming polymer. If it is less than 0.01 wt. %, or more than 10 wt. %, the plasticizing action may be impaired.

In an embodiment of the invention, said hard capsule shell film and thereby said hard capsule shell consist of the film forming polymer, magnesium oxide and plasticizer only, except for water in form of residual moisture

In case that said hard capsule shell film and thereby said hard capsule shell consist of the film forming polymer, magnesium oxide and plasticizer only, except for water in form of residual moisture, possible ranges for film forming polymer, magnesium oxide and plasticizer based on weight of dry film or dry capsule shell respectively are:
from 76.92 to 99.74 wt.% of film forming polymer,
from 0.25 to 15.38 wt.% of magnesium oxide and
from 0.01 to 7.69 wt.% of plasticizer; preferably
from 86.96 to 97.94 wt.% of film forming polymer,
from 1.96 to 8.70 wt.% of magnesium oxide and
from 0.05 to 4.35 wt.% of plasticizer; more preferably
from 91.32 to 94.97 wt.% of film forming polymer,
from 4.75 to 7.31 wt.% of magnesium oxide and
from 0.28 to 1.37 wt.% of plasticizer.

The hard capsule shell film of the present invention may comprise a gelling agent, a gelling aid, a pH adjuster, a sweetener, an acidulant, a preservative, and a fragrance, a binder, a thickener, a colorant other than magnesium oxide and the like, depending on the type of film forming polymer used and the purpose of use.

Colorants are usually distinguished as being either dyes or pigments, with dyes being defined as colorants which are soluble in the application media, that is they are soluble in the film forming polymer solution of the hard capsule shell film, whereas pigments are defined as such colorants which are insoluble in the application media, that is in the film forming polymer solution of the hard capsule shell film.

Any colorant comprised in the hard capsule shell film has preferably a color other than white, so in an embodiment, the hard capsule shell film is free from the white-color colorant titanium oxide;
in an embodiment, the hard capsule shell film is free from titanium oxide;
in an embodiment, the hard capsule shell film is free from the white-color colorant calcium carbonate;
in an embodiment, the hard capsule shell film is free from calcium carbonate;
in an embodiment, the hard capsule shell film is free from the white-color colorant titanium oxide and is free from the white-color colorant calcium carbonate;
in an embodiment, the hard capsule shell film is free from titanium oxide and is free from calcium carbonate;
in an embodiment, the hard capsule shell film is free from a colorant with a white color (also called a white-color colorant) other than magnesium oxide.

In case of a colorant the hard capsule shell film may comprise one or more colorants other than magnesium oxide, preferably any such colorant has a color other than white; said colorant or mixture of such colorants may be used for coloring the hard capsule shell film with, for example, with a black, grey or chromatic color. Chromatic colors refer to all colors other than black, white, and gray.

In a case of using colorants for pharmaceutical preparations or health food preparations, the colorants may be used as long as such colorants are allowed to be used in the field of pharmaceuticals and food. As examples for such colorants tar colorant lakes, water-soluble tar colorants and natural colorant, caramel, cochineal colorants, lac colorants, riboflavin, sodium iron chlorophyllin, sodium copper chlorophyllin, copper chlorophyll, iron sesquioxide, iron black oxide and the like can be mentioned. For the purpose of giving pearl luster, pearl pigments made from appropriate amount of fine powder such as a fish scale foil, nacre or mica of shellfish, silicon dioxide, and the like can be used. In a preferred embodiment, magnesium oxide is the only component in the hard capsule shell film which provides for a white color.
In a preferred embodiment, the hard capsule shell film comprises no other component that provides for a white color.

In an embodiment of the invention, said hard capsule shell film and thereby said hard capsule shell consist of the film forming polymer and magnesium oxide, and optionally a plasticizer and optionally a colorant other than a white color colorant, only, except for water in form of residual moisture.

In case that said hard capsule shell film and thereby said hard capsule shell consist of the film forming polymer and magnesium oxide, and optionally a plasticizer and optionally a colorant other than a white color colorant, only, except for water in form of residual moisture, said hard capsule shell film and thereby said hard capsule shell preferably contain, based on weight of dry film or dry capsule shell respectively,
at least 75 wt.% of film forming polymer,
at least 1 wt.% of magnesium oxide
optionally at least 0.1 wt.% of plasticizer;
optionally at least 0.5 wt.% of colorant other than white color colorant; with the amounts of film forming polymer, magnesium oxide, optionally plasticizer and optionally colorant other than white colorant adding up to 100 wt.%; with the film forming polymer, magnesium oxide, plasticizer and white color colorant as defined herein, also with all their embodiments.

Some examples of the gelling agent used in the film forming composition of the present invention include carrageenan, gellan gum, agar, pectin, gelatin, xanthan gum, locust bean gum, curdlan, sodium alginate, glucomannan, tamarind seed gum, and methyl cellulose. However, they are not limited to the above.

The amount of the gelling agent may be up to 10 wt. %, preferably up to 5 wt. %, with the wt. % being based on the amount (dry weight) of a film forming polymer.

Examples of the gelling aid used in the film forming composition of the present invention include sodium salts, potassium salts, calcium salts and the like suitable for the gelling agent to be used. These may be used alone or in combination in appropriate amounts. The amount of the gelling aid may be up to 10 wt. %, preferably up to 5 wt. %, with the wt. % being based on the amount (dry weight) of a film forming polymer.

Thickeners include alginic acid, furcelleran, taragum, karaya gum, succinoglycan, xanthan gum, gum arabic, karaya gum, guar gum, tamarind seed gum, tara gum, tragant gum, CMC-Ca, CMC-Na, glucosamine, pullulan, sodium polyacrylate, curdlan, and modified starch. A thickener or a binder can increase gel strength and supplement the strength of the film or shell.

Further subject of the invention is a hard capsule shell wherein the shell is formed by a hard capsule shell film as defined herein, also with all its embodiment.

Further subject of the invention is a method for preparing a hard capsule shell, wherein the hard capsule shell is prepared by dip molding and with the shell of the hard capsule shell being formed by a hard capsule shell film, with the hard capsule shell film as defined herein, also with all its embodiments.

The hard capsule shell according to the present invention can be manufactured by any dip molding method which is known for the preparation of a hard capsule shell. As an example, the case of gelatin capsules will be described.

Firstly, 20 to 40 parts by weight of gelatin is homogeneously dissolved in hot water to prepare a pale yellow and transparent immersion liquid derived from gelatin color. A dispersion of magnesium oxide and optionally an aqueous solution of the plasticizer are added to the immersion liquid. After adjusting the viscosity of the liquid, a stainless-steel molding pin is dipped in to the liquid and subsequently withdrawn out of the liquid. The immersion liquid adhering around the pulled-up molding pin immediately is cooled and gelled to secure a film thickness suitable for a capsule. After drying, unnecessary parts are cut and a capsule is manufactured by fitting a pair of caps with a body.

Some examples of drying conditions include high-temperature drying in an oven at a temperature of 60 °C for 15 minutes, high-temperature drying in an oven at a temperature of 60 °C for 30 minutes, and natural drying at room temperature.

A hard capsule shell can comprise one or more layers of films of polymers, preferably one or more layers of films of film-forming polymers. For ease of reading such a layer of a film of polymer is simply called layer herein. For a definition of a layer in the sense of this invention and in case of more than one layer, each layer is made in an own process step which is separate from a process step making another layer.

Typical process steps for preparing a layer can be dip molding, banding or coating, such as spray coating.

In case of a hard capsule shell with only one layer, this one layer is the hard capsule shell film as it is described herein; that means the hard capsule shell has no further layer; that means for example it is not banded or coated but has only said one layer.

Preferably, in case of a hard capsule shell with only one layer, this one layer is said hard capsule shell film and this one layer is formed by dip molding.

In case of a hard capsule shell with more than one layer, then at least one layer different from the outermost layer, preferably the innermost layer, is said hard capsule shell film as it is described herein, preferably it is formed by dip molding.

In case of a hard capsule shell with more than one layer, then preferably the hard capsule shell has 2, 3, 4 or 5 layers. In case of more than one layer, the compositions of the layers can be identical of different, preferably the compositions of the layers which are in touch with each other are different. In a particular embodiment all layers have different compositions from each other.

Preferably in case of more than one layer, at least the innermost layer is formed by dip molding.

In an embodiment, the hard capsule shell has two layers, with the inner layer being said hard capsule shell film, preferably with said hard capsule shell film having been prepared by dip molding.

In an embodiment, the hard capsule shell has two layers, with the inner layer being said hard capsule shell film and with the outer layer being a coating or a banding; preferably with said hard capsule shell film having been prepared by dip molding.

In an embodiment, the hard capsule shell has two layers, with the inner layer being said hard capsule shell film and with the outer layer having been prepared by dip molding, preferably with said hard capsule shell film having been prepared by dip molding.

In an embodiment, the hard capsule shell has two layers, with the inner layer being said hard capsule shell film and with the outer layer not being a banding or a coating, preferably with said hard capsule shell film having been prepared by dip molding.

In an embodiment, the hard capsule shell has two layers, with the inner layer said hard capsule shell film and with the outer layer not having been prepared by dip molding, preferably with said hard capsule shell film prepared by dip molding.

In any of the mentioned embodiments where the hard capsules shell has two layers, the outer layer can contain magnesium oxide; preferably the outer layer can contain magnesium oxide as whitening agent.

In any of the mentioned embodiments where the hard capsules shell has two layers, the outer layer may be free of magnesium oxide as whitening agent.

In any of the mentioned embodiments where the hard capsules shell has two layers, the outer layer may be free of magnesium oxide.

In an embodiment, if the hard capsule shell contains more than two layers, then at least one of the layers different from the outermost layer, preferably at least the innermost layer, is said hard capsule shell film and at least one of the other layers can contain magnesium oxide, preferably as whitening agent.

In an embodiment, if the hard capsule shell contains more than two layers, then at least one of the layers different from the outermost layer, preferably at least the innermost layer, is said hard capsule shell film and at least one of the other layers, preferably the outermost layer, may be free of magnesium oxide as whitening agent.

In an embodiment, if the hard capsule shell contains more than two layers, then at least one of the layers different from the outermost layer, preferably at least the innermost layer, is said hard capsule shell film and at least one of the other layers, preferably the outermost layer, may be free of magnesium oxide.

### [Effect of the invention]

According to the present invention, materials filled in a hard capsule can be shielded from light without using titanium oxide which is a concern for carcinogenicity by using magnesium oxide which is not harmful to health and conventionally used in the pharmaceuticals and food fields. Since magnesium oxide has a light-shielding property comparable to that of titanium oxide, it can be suitably used not only for normal whitening of the capsule but also for formulation of light-unstable pharmaceuticals and food.

Further, the light-shielding hard capsule shell of the present invention can maintain stable light-shielding property for a long period of time without lowering the solubility even if it contains magnesium oxide. Furthermore, when used in combination with a chromatic colorant such as a dye or a pigment, an opaque and chromatically colored film can be obtained.

Therefore, especially for the health food market, it is possible to provide a safe and beautiful colored light-shielding capsule shell by combining with natural colorants such as gardenia.

Further embodiments of the invention are:
(A) A hard capsule shell film comprising a film forming polymer and magnesium oxide, wherein said hard capsule shell film is free from the white-color colorant titanium oxide.
(B) A hard capsule shell film according to embodiment (A), wherein said hard capsule shell film is free from the white-color colorant calcium carbonate.
(C) A hard capsule shell film according to embodiment (A) or (B), wherein said hard capsule shell film is free from a white-color colorant other than magnesium oxide.
(D) The hard capsule shell film according to anyone of embodiments (A) to (C), wherein the particle size of magnesium oxide is 50 um or less.
(E) The hard capsule shell film according to anyone of embodiments (A) to (D), wherein the magnesium oxide has a particle size distribution with a D10 value of from 0.1 to 2.0 um.
(F) The hard capsule shell film according to anyone of embodiments (A) to (E), wherein the magnesium oxide has a particle size distribution with a D90 value of from 4 to 45 um.
(G) The hard capsule shell film according to any one of embodiment (A) to (F), wherein the amount of film forming polymer is at least 75% wt., with the wt.% being based on the dry weight of the hard capsule shell film.
(H) The hard capsule shell film according to any one of embodiments (A) to (G), wherein the magnesium oxide is heavy magnesium oxide.
(I) The hard capsule shell film according to any one of embodiments (A) to (H), wherein the amount of magnesium oxide is from 0.25 to 20 wt. %, with the wt.% being based on the dry weight of the film forming polymer.
(J) The hard capsule shell film according to any one of embodiments (A) to (I), wherein the amount of magnesium oxide is from 2 to 10 wt. %, with the wt.% being based on the dry weight of the film forming polymer.
(K) The hard capsule shell film according to any one of embodiments (A) to (J), wherein the amount of magnesium oxide is from 5 to 8 wt. %, with the wt.% being based on the dry weight of the film forming polymer.
(L) The hard capsule shell film according to any one of embodiments (A) to (K), wherein the film forming polymer is selected from the group consisting of gelatin, polyglutamic acid, cellulose derivative, pullulan, starch, dextrin, agar, chitosan, acid-treated crushed yeast, polyvinyl alcohol (PVA), PVA-methylmethacrylate copolymer, polyvinylpyrrolidone (PVP).
(M) The hard capsule shell film according to any one of embodiments (A) to (L), further comprising a plasticizer.
(N) The hard capsule shell film according to embodiment (M), wherein the plasticizer is selected from the group consisting of sorbitan fatty acid ester, triethyl citrate, glycerin, sorbitol, mannitol, trehalose, vegetable oil, medium chain fatty acid triglyceride, triacetin, phthalates, phytosterol, propylene glycol, polysorbate and polyethylene glycol.
(O) The hard capsule shell film according to embodiment (M) or (N), wherein the amount of the plasticizer is from 0.01 to 10 wt. %, with the wt.% being based on the dry weight of the film forming polymer.
(P) The hard capsule shell film according to embodiment (N), wherein the plasticizer is sorbitan fatty acid ester.
(Q) The hard capsule shell film according to embodiment (P), wherein the plasticizer is sorbitan monolaurate.
(R) The hard capsule shell film according to any one of embodiments (A) to (Q), which is colored by adding a colorant wherein the colorant has a color other than white.
(S) A hard capsule shell wherein the shell is formed by the hard capsule shell film according to any one of embodiment (A) to (R).
(T) A method for preparing a hard capsule shell according to embodiment (S), wherein the hard capsule shell is prepared by dip molding.

### [Simple explanation of drawings]

[FIG. 1] FIG. 1 shows the particle size distribution of food additive grade magnesium oxide.
[FIG. 2] FIG. 2A shows the light transmittance of each film obtained from HPMC solution wherein the rage of 0 to 20 wt. % magnesium oxide is dispersed. FIG. 2B are photographs of the films of which light transmittance were measured.
[FIG. 3] FIG. 3 shows the light transmittance of each film obtained from HPMC solution wherein the rage of 0 to 15 wt. % magnesium oxide is dispersed.
[FIG.4] FIG. 4A shows the light transmittance of a film obtained from a gelatin solution in which 10% magnesium oxide is dispersed with respect to the amount of gelatin. FIG. 4B are photographs of films of which light transmission was measured.

### <Examples>

### (A) Analysis of magnesium oxide particle size

Magnesium oxide was dispersed with sorbitan monolaurate in ion exchanged water at a maximum speed of 6600 rpm for 15 minutes using IKA's T50 ULTRA TURRAX to provide a dispersion containing magnesium oxide 40 wt. %, SML 2.7 wt.% and ion exchanged water 57.3 wt.%. As the magnesium oxide, "MgO, Food grade" from TOMITA Pharmaceutical CO. ,Ltd.(MS-0) was used. Calcining temperature of MS-0 is 600°C.

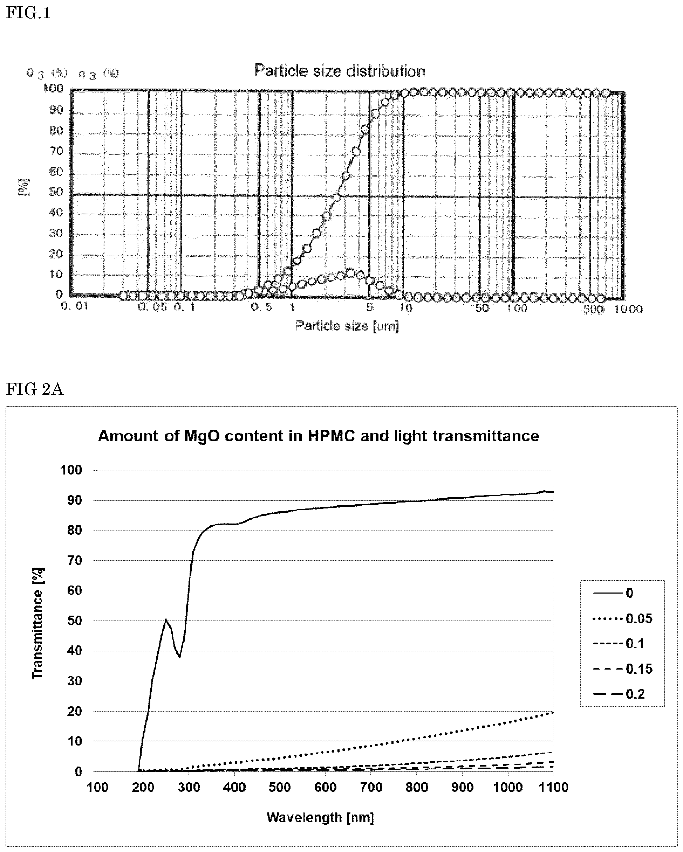

The required amount of the dispersion prepared above was added dropwise to ion exchanged water. The particle size distribution was measured with the Shimadzu SALD-2000J laser reflection type particle size distribution measuring instrument at a wave length of 680 nm.
The values of D10, D50, and D90 were 0.801, 2.577, and 5.739, respectively (FIG. 1).

In FIG. 1 it can be seen that the particle sizes are less than 50 µm, less than 45 µm, less than 40 µm, less than 35 µm, and less than 30 µm.

### (B) Analysis of film elongation rate

By adding a 40 wt.% aqueous dispersion of magnesium oxide (MS-0) at room temperature to an aqueous solution of hypromellose (HPMC2906) at room temperature, three kinds of solutions were prepared in which the concentration of hypromellose (solid content) was 20 wt. % and the ratio of magnesium oxide to the weight of hypromellose was 0.5, 7.5, and 15 wt. % magnesium oxide was used.

As the hypromellose, HPMC 2906 manufactured by Shin-Etsu Chemical Co., Ltd. was used in the test. After stirring the dispersion well to defoam, a film was made using CAMAG film applicator in the following manner: A 20x20x0.3 cm glass plate was pre-warmed to 70 °C, and with the film applicator, the film was pulled to a thickness of 100 um when dried, and immediately placed in a 60 °C oven to dry for 15 minutes.

The films were cut out into strips measuring 1 cm in length and 10 cm in width, and stored in a moisture control box with a relative humidity of 2.5% RH for 7 days to adjust the moisture content. After that, the film was pulled at a speed of 5 mm / min using a universal testing machine model 68TM-5 manufactured by INSTRON, and the elongation % of the film until the film was cut was measured (Table 3). It was found that the elongation rate of the film decreased as the amount of magnesium oxide increased.

**[Table 3-1]**

| MS-0 | | | |
|---|---|---|---|
| Amount of MgO [wt. % against polymer wt.] | 0.5 | 7.5 | 15 |
| Film elongation rate [%] | 13. 0 | 8. 4 | 3. 9 |

Using MS-2 as the magnesium oxide, the elongation rate of the film was measured in the same manner as above.

**[Table 3-2]**

| MS-2 | | | |
|---|---|---|---|
| Amount of MgO [wt. % against polymer wt.] | 0.5 | 7.5 | 15 |
| Film elongation rate [%] | 16.0 | 12.2 | 7.7 |

The film comprising MS-2 has higher film elongation rate than MS-0, which indicates that MS-2 brought better elongation.

In the following examples (C) to (D) and (F), the same hypromellose as used in (B) was used. In the following examples (C) to (E), as the magnesium oxide, MS-0 and MS-2 were used. In (F), MS-0 was used.

### (C) Action of a plasticizer

Five types of films comprising sorbitan monolaurate (SML; CAS1338-39-2), in the ratio shown in Table 2 with respect to the weight of hypromellose were prepared, using a dispersion containing 0.5, 7.5, and 15 wt. % of magnesium oxide with respect to hypromellose, the dispersions were prepared with a 40 wt.% aqueous dispersion of magnesium oxide (MS-0). After drying and adjusting the water content by the same manner as in (B), the film elongation rate was measured with the same universal tester (Table 4).

**[Table 4-1]**

| MS-0 | | | | | |
|---|---|---|---|---|---|
| MgO [wt.% against Polymer wt.] | 0.5 | 7.5 | 7.5 | 15 | 15 |
| SML [wt.% against Polymer wt.] | 0.10 | 0.10 | 1.00 | 0.10 | 1.0 |
| Film elongation rate [%] | 14. 7 | 9. 0 | 9. 1 | 4. 4 | 6. 3 |

As a result, it was confirmed that the addition of SML made the film flexible and increased the elongation rate, compared with Table 3. This suggests that SML acts as a plasticizer. It was confirmed that the addition of the plasticizer improved the decrease in the plasticity of the film due to magnesium oxide. Empirically, the higher the elongation rate of the film, the less likely the capsule will crack due to impact.

Using MS-2 as the magnesium oxide, the elongation rate of the film was measured in the same manner as above.

**[Table4-2]**

| MS-2 | | | | | |
|---|---|---|---|---|---|
| MgO [wt.% against Polymer wt.] | 0.5 | 7.5 | 7.5 | 15 | 15 |
| SML [wt.% against Polymer wt.] | 0.1 | 0.1 | 1 | 0.1 | 1 |
| Film elongation rate [%] | 16.2 | 14.0 | 11.1 | 10.4 | 8.6 |

Even if magnesium oxide (MS-0) was replaced with MS-2, sorbitan monolaurate (SML) acted as a plasticizer and MS-2 & SML gave a higher elongation rate than MS-0 & SML.

### (D) Light-shielding effect of magnesium oxide

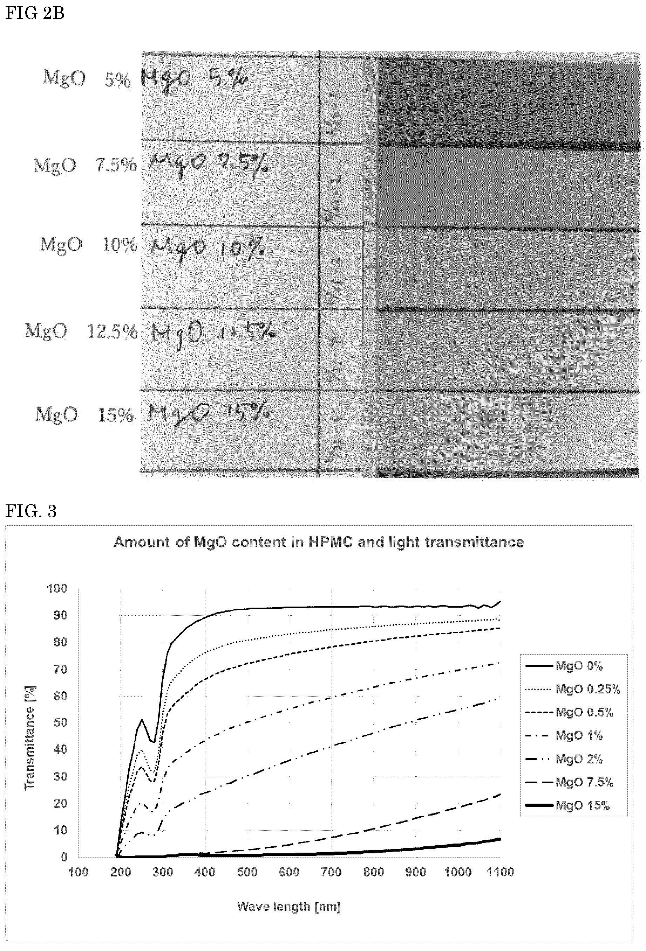

Sample films were produced in the same manner as in (B) by adding 5%, 10%, 15%, and 20 wt. % of magnesium oxide (MS-0) to the hypromellose solution with respect to the weight of hypromellose. A film without magnesium oxide was prepared, too. Light transmittances at wavelengths from 190 nm to 1100 nm were measured by UV-1900i manufactured by Shimadzu Corporation (FIGs. 2A, 2B and Table 5).

Similarly, 0.25%, 0.5, 1, 2, 7.5 and 15 wt. % of magnesium oxide with respect to the weight of hypromellose was suspended in a hypromellose solution, and sample films were produced in the same manner as in (B). The light transmittance was measured for the films, too (FIG. 3, Table 5).

**[Table 5-1]**

| MS-0 | | | | | |
|---|---|---|---|---|---|
| | 200nm | 400nm | 600nm | 800nm | 1100nm |
| MgO 0% | 11.5 | 82.1 | 87.7 | 89.7 | 92.9 |
| MgO 0.25% | 9.9 | 76.1 | 83.1 | 85.9 | 88.2 |
| MgO 0.5% | 8.7 | 66.3 | 75.6 | 80.5 | 85.2 |
| MgO 1% | 5.2 | 43.5 | 55.3 | 63.3 | 72.5 |
| MgO 2% | 2.2 | 23.9 | 35.9 | 46.3 | 59 |
| MgO 5% | 0.1 | 2.9 | 6.4 | 10.9 | 19.6 |
| MgO 7.5% | 0 | 1.3 | 3.0 | 5.8 | 12.3 |
| MgO 10% | 0 | 0.6 | 1.4 | 2.7 | 6.3 |
| MgO 13% | 0 | 0.6 | 1.0 | 1.8 | 4.4 |
| MgO 15% | 0 | 0.5 | 0.8 | 1.3 | 3.0 |
| MgO 20% | 0 | 0.3 | 0.5 | 0.7 | 1.7 |

It was found that even if only 0.25 wt. % of magnesium oxide was dispersed in hypromellose, the light was shielded as compared with the case where no magnesium oxide was added.

Using MS-2 as the magnesium oxide, the light transmittance was measured in the same manner as above.

**[Table 5-2]**

| MS-2 | | | | | |
|---|---|---|---|---|---|
| | 200nm | 400nm | 600nm | 800nm | 1100nm |
| MgO 0% | 11.5 | 82.1 | 87.7 | 89.7 | 92.9 |
| MgO 0.25% | 3.7 | 57.5 | 74.5 | 82.0 | 87.0 |
| MgO 0.5% | 2.1 | 43.3 | 65.0 | 76.1 | 84.5 |
| MgO 1% | 0.7 | 20.0 | 42.7 | 59.6 | 74.0 |
| MgO 2% | 0.1 | 6.2 | 21.7 | 40.0 | 60.5 |
| MgO 5% | 0 | 0.9 | 2.7 | 9.1 | 26.5 |
| MgO 7.5% | 0 | 0.6 | 1.1 | 3.8 | 16.2 |
| MgO 10% | 0 | 0.5 | 0.8 | 1.5 | 7.5 |
| MgO 13% | 0 | 0.5 | 0.7 | 0.9 | 4.1 |
| MgO 15% | 0 | 0.5 | 0.6 | 0.8 | 2.8 |
| MgO 20% | 0 | 0.4 | 0.6 | 0.6 | 1.3 |

At least at visible wavelengths of 400/600/800, the light transmittance of MS-2 was lower than that of MS-0, indicating MS-2 had better light blocking performance.

### (E) Light-shielding effect of magnesium oxide in Gelatin film

Using porcine skin gelatin (acid-treated gelatin, A type / gelatin) manufactured by Lucero and bovine bone gelatin (alkali-treated gelatin, B type / gelatin) manufactured by Nitta Gelatin, 30 wt.% gelatin aqueous solution at 50 ° C was prepared.

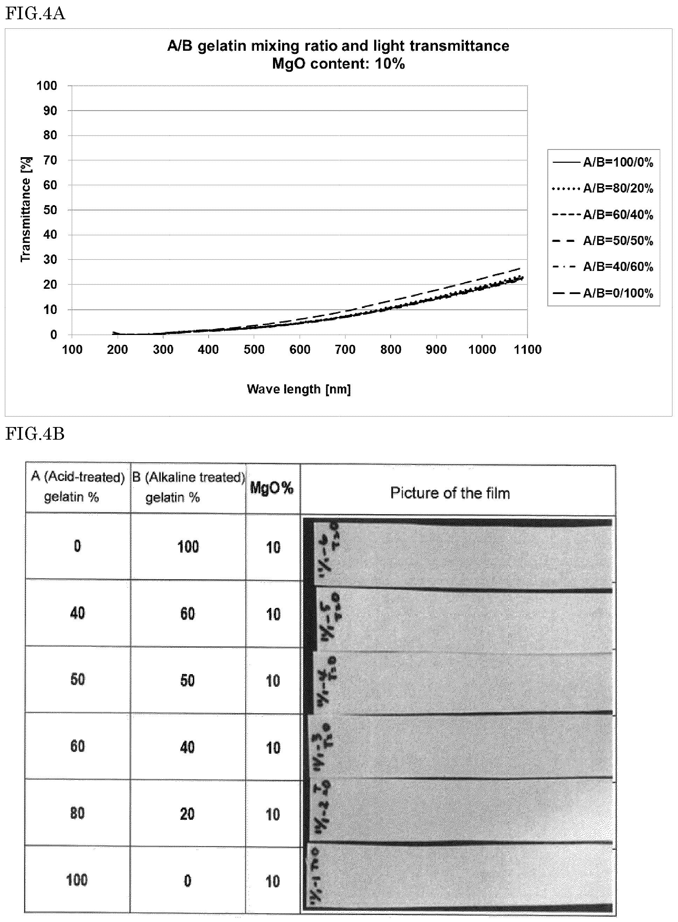

As the gelatin, 6 types having a weight ratio A type/B type of 100/0, 80/20, 60/40, 50/50, 40/60 and 0/100% of the above two types of gelatin were used. The magnesium oxide (MS-0) dispersion was added to each gelatin solution in an amount of 10 wt. % based on the weight of gelatin, and the mixture was stirred well to defoam. A film having a thickness of 100 um after drying was prepared from this dispersion using a film making machine (FIG. 4B). The light transmittance of the film was measured by the same method as in (D). (FIG. 4A, Table 6)

**[Table 6-1]**

| MS-0 | | | | | |
|---|---|---|---|---|---|
| | 200 nm | 400 nm | 600 nm | 800 nm | 1100 nm |
| A/B=100/0% | 0 | 1.8 | 5.0 | 10.8 | 22.4 |
| A/B=80/20% | 0 | 1.7 | 5.0 | 11.3 | 23.7 |
| A/B=60/40% | 0 | 1.7 | 4.9 | 11.0 | 23.1 |
| A/B=50/50% | 0 | 1.6 | 4.8 | 10.8 | 22.7 |
| A/B=40/60% | 0 | 1.7 | 4.7 | 10.6 | 22.1 |
| A/B=0/100% | 0 | 2.1 | 6.5 | 13.9 | 26.8 |

Using MS-2 as the magnesium oxide, the light transmittance was measured in the same manner as above.

**[Table 6-2]**

| MS-2 | | | | | |
|---|---|---|---|---|---|
| | 200 nm | 400 nm | 600 nm | 800 nm | 1100 nm |
| A/B=100/0% | 0 | 0.7 | 1.5 | 4.9 | 17.1 |
| A/B=80/20% | 0 | 0.7 | 2.0 | 7.7 | 24.4 |
| A/B=60/40% | 0 | 0.7 | 1.9 | 7.4 | 24.5 |
| A/B=50/50% | 0 | 0.8 | 2.6 | 9.6 | 27.6 |
| A/B=40/60% | 0 | 0.8 | 2.8 | 9.9 | 27.9 |
| A/B=0/100% | 0 | 0.9 | 3.7 | 12.4 | 31.7 |

At least at visible wavelengths of 400/600/800, the light transmittance of MS-2 is lower than that of MS-0, indicating MS-2 has better light blocking performance.

### (F) Elution test

Using a 40 wt. % aqueous dispersion of magnesium oxide, a dispersion containing 20 wt. % hypromellose , 7.5 wt. % of magnesium oxide and 0.5 wt. % of SML with respect to the weight of hypromellose was prepared. Using a hard capsule manufacturing machine (made by CAPSUGEL), a size 1 hypromellose capsule was manufactured by immersing a metal mold of a capsule heated to 80 ° C in the dispersion to gel hypromellose. This capsule was filled with 280 mg of acetaminophen powder, and the elution test was carried out using a model NTR-6300A elution tester manufactured by Toyama Sangyo under the conditions of a USP pH 1.2 solution at 37 ° C, with a sinker, and a paddle rotation speed of 50 rpm. As a reference, a size 1 hypromellose capsule free from magnesium oxide was produced under the same production conditions as the above.
The average elution rates of 6 capsules were as follows (Table 7).

**[Table 7]**

| | | | | | | |
|---|---|---|---|---|---|---|
| Elution time [min] | | 0 | 15 | 30 | 45 | 75 |
| Elution rate of Acetaminophen [%] | MgO 0% | 0 | 33 | 78 | 92 | 100 |
| | MgO 7.5% | 0 | 50 | 73 | 90 | 100 |

It was confirmed that no decrease in elution was observed in the hypromellose capsules comprising magnesium oxide.

### [Industrial applicability]

According to the present invention, instead of titanium oxide which is considered to have a health problem, magnesium oxide can impart light-shielding properties to capsule shells as a light-shielding agent. Magnesium oxide has been safely used as a pharmaceutical product. The light-shielding capsule obtained by the present invention retains solubility and maintains stable light-shielding property for a long period of time. Moreover, it can give an opaque colored film when used together with a dye. Accordingly, magnesium oxide is very useful and safe to manufacture for a capsule for filling pharmaceuticals, veterinary drugs, pesticides, cosmetics, health food, etc.

## Claims

1. A hard capsule shell wherein the shell is formed by a hard capsule shell film, the hard capsule shell film comprising a film forming polymer and magnesium oxide,
wherein said hard capsule shell film is free from the white-color colorant titanium oxide; and
wherein the hard capsule shell has only one layer, this one layer is said hard capsule shell film, or
the hard capsule shell has more than one layer and at least one layer different from the outermost layer is said hard capsule shell film.

2. A hard capsule shell according to claim 1, wherein said hard capsule shell film is free from the white-color colorant calcium carbonate.

3. A hard capsule shell according to claim 1 or 2, wherein said hard capsule shell film is free from a white-color colorant other than magnesium oxide.

4. The hard capsule shell according to anyone of claims 1 to 3, wherein the particle size of magnesium oxide is 50 um or less.

5. The hard capsule shell according to anyone of claims 1 to 4, wherein the magnesium oxide has a particle size distribution with a D10 value of from 0.1 to 2.0 um.

6. The hard capsule shell according to anyone of claims 1 to 5, wherein the magnesium oxide has a particle size distribution with a D90 value of from 4 to 45 um.

7. The hard capsule shell according to any one of claims 1 to 6, wherein the amount of film forming polymer is at least 75 wt. %, with the wt. % being based on the dry weight of the hard capsule shell film.

8. The hard capsule shell according to any one of claims 1 to 7, wherein the magnesium oxide is heavy magnesium oxide.

9. The hard capsule shell according to any one of claims 1 to 8, wherein the amount of magnesium oxide is from 0.25 to 20 wt. %, with the wt. % being based on the dry weight of the film forming polymer.

10. The hard capsule shell according to any one of claims 1 to 9, wherein the amount of magnesium oxide is from 2 to 10 wt. %, with the wt. % being based on the dry weight of the film forming polymer.

11. The hard capsule shell according to any one of claims 1 to 10, wherein the amount of magnesium oxide is from 5 to 8 wt. %, with the wt. % being based on the dry weight of the film forming polymer.

12. The hard capsule shell according to any one of claims 1 to 11, wherein the film forming polymer is selected from the group consisting of gelatin, polyglutamic acid, cellulose derivative, pullulan, starch, dextrin, agar, chitosan, acid-treated crushed yeast, polyvinyl alcohol (PVA), PVA-methylmethacrylate copolymer, polyvinylpyrrolidone (PVP).

13. The hard capsule shell according to any one of claims 1 to 12, further comprising a plasticizer.

14. The hard capsule shell according to claim 13, wherein the plasticizer is selected from the group consisting of xylitol, fructose, sorbitan fatty acid ester, triethyl citrate, glycerin, sorbitol, mannitol, trehalose, vegetable oil, medium chain fatty acid triglyceride, triacetin, phthalates, phytosterol, propylene glycol, polysorbate and polyethylene glycol.

15. The hard capsule shell according to claim 13 or 14, wherein the amount of the plasticizer is from 0.01 to 10 wt. %, with the wt.% being based on the dry weight of the film forming polymer.

16. The hard capsule shell according to claim 14, wherein the plasticizer is sorbitan fatty acid ester.

17. The hard capsule shell according to claim 16, wherein the plasticizer is sorbitan monolaurate.

18. The hard capsule shell according to any one of claims 1 to 17, which is colored by adding a colorant wherein the colorant have a color other than white.

19. A method for preparing a hard capsule shell according to claim 1, wherein said hard capsule shell film comprising a film forming polymer and magnesium oxide is prepared by dip molding
